# EUROPEAN PATENT SPECIFICATION

(11) **EP 4 061 474 B1**
(45) Date of publication and mention of the grant of the patent: **01.05.2024**
(21) Application number: 20829006.4
(22) Date of filing: 18.11.2020
(51) Int. Cl.: A61N 1/05, A61B 5/291

(54) **ELECTRODE FOR RECORDING ELECTROENCEPHALOGRAPHIC SIGNALS AND/OR STIMULATING PATIENTS**
ELEKTRODE ZUR AUFZEICHNUNG VON ELEKTROENCEPHALOGRAPHISCHEN SIGNALEN UND/ODER ZUR STIMULATION VON PATIENTEN
ÉLECTRODE POUR L'ENREGISTREMENT DE SIGNAUX ÉLECTRO-ENCÉPHALOGRAPHIQUES ET/OU LA STIMULATION DE PATIENTS

(30) Priority: 19.11.2019 IT 201900021561
(43) Date of publication of application: 28.09.2022
(73) Proprietor: Spes Medica SRL, 16153 Genova (IT)
(72) Inventor: MISTRETTA, Matteo, 16153 Genova (GE) (IT); PHELAN, Patrick, 16153 Genova (GE) (IT)
(74) Representative: Bottino, Giovanni
(86) International application number: PCT/IB2020/060852
(87) International publication number: WO 2021/099952

(56) References cited:
- CN-A- 104 068 853
- US-A- 3 845 757
- US-A- 5 785 040
- US-A1- 2015 238 106
- US-A1- 2017 079 543
- US-A1- 2017 164 860

## Description

The present invention relates to an electrode for recording electroencephalographic signals and/or stimulating patients according to claim 1.

The electrode comprises a patient contact part and a transmission part.

A layer of electrolyte material is further provided in contact with the patient's skin and interposed between the patient's skin and the contact part.

That just described is the common configuration of electrodes used for the detection of extracranial electroencephalographic (EEG) traces, i.e., which include the application of electrodes in contact with the patient's skin.

As known in state-of-the-art electrodes, the contact part is placed in contact with the patient's skin, through a layer of electrolyte material and deals with the transduction of signals detected at the skin level and the generation of electrical signals which are transmitted, through the transmission part, to an external unit, aimed at generating the EEG traces.

An electrode belonging to the state of the art is disclosed in document CN104068853, in which a bioelectric electrode is extensively applied to bioelectric recording, measurement and stimulation, including high-density electrode measurement, medical facilities, mobile equipments, family health care, psychological cognition, games, a brain-computer interface, rehabilitation training and the like, and is particularly applicable to electroencephalogram measurement.

Another solution belonging to the state of the art is disclosed in the document US2017164860, describing a long term physiological signal sensing patch, including a water protective film, an electrode layer, a conductive hydrogel layer, a block layer and a first attaching layer.

The document US5785040 relates to a medical electrode system including an electrically non-conductive backing material and a flexible, electrically conductive patch disposed on the backing material. An electrically non-conductive facing material is disposed over the patch which includes an aperture therein for exposing a portion of the patch, said aperture having a maximum dimension smaller than a minimum dimension of the patch.

Document US3845757 refers to a pregelled intensive care electrode formed from two layers of soft, conformable foam material having a rigid plastic supporting layer between the foam layers. A conductive connector is in contact with an open-celled spongy material filled with an electrolyte which provides means for connecting the electrode to monitoring devices and measuring the electrical impulses from a patient's skin. Another prior art electrode is known from CN 104 068 853.

One of the possible uses of the state-of-the-art electrodes is the monitoring of patients for abnormalities in EEG traces and the surveillance of diseases, such as epilepsy.

In order to obtain relevant results and correctly identify anomalies in EEG pathways, it is necessary to foresee long and continuous monitoring periods, as the longer the monitoring time, the greater the information available to the doctor, in order to identify, for example, the timing of epileptic seizures, the epileptic focus or to detect the effectiveness of certain drugs.

This monitoring can therefore vary from a few hours to one or more weeks, during which time the patient is "connected" to the electroencephalographic equipment, i.e., to the electrode system and external unit.

For this reason, increasingly lightweight and portable external units have been developed, which can be easily fixed to the patient's body, so that the weight thereof does not hinder the patient's movements during the monitoring.

Although the development of external units allows long-term monitoring, the state-of-the-art systems have particularly disadvantageous aspects related to the electrodes attached to the patient's skin, which at the moment have been developed for short-term monitoring.

In fact, the electrodes known in the state of the art, for long-term monitoring, generate a pressure action on the patient's skin, risking causing trauma to the patient's skin due to so-called "skin breakdown".

Skin breakdown is a phenomenon which involves the formation of sores in the patient's skin, created due to the wearing of the skin in contact with the electrode, sores which form over the long term due to the pressure exerted by the electrodes and which become infected due to the presence of the layer of electrolyte material in contact with the hair and sebum of the patient's skin.

The "skin breakdown" phenomenon is particularly critical for the patient and requires specific treatments, causing problems for patients who already have serious clinical conditions.

The pressure exerted by the electrode is caused by the specific creation of the electrodes known in the state of the art, the contact part of which consists of conductive material, generally conductive rigid plastic material or metallic material.

The contact parts of the electrodes known in the state of the art are therefore made of rigid materials: rigidity is a fundamental aspect since the electrode must deal with the transduction of an ionic signal to an electrical signal, so it must guarantee perfect contact and not have movements which can cause signal distortions.

However, the characteristic rigidity of the materials currently used is precisely the cause of the manifestation of the "skin breakdown".

There is therefore a need not satisfied by the systems known in the state of the art, to create an electrode which solves the disadvantages described above, in particular which avoids the onset of "skin breakdown".

The present invention achieves the above objects by providing an electrode as described above, and according to the characterizing part of claim 1.

An electrode is thus created, the prolonged application of which on the patient's skin does not generate deterioration of the skin itself, thanks to the softness and flexibility of the materials, which allow a positioning of the electrode without causing trauma to the patient.

In order to obtain an electrode having certain flexibility and softness features, the present invention includes two main solutions.

According to a first embodiment, the contact part comprises a cup element adapted to house at least in part the layer of electrolyte material.

The cup element consists of a matrix of rubbery material, inside which matrix particles of conductive material are embedded.

This solution therefore involves creating a soft and deformable conduction part, but which has the same electrical features as conductive rigid plastic.

In fact, the presence of conductive particles allows to obtain a deformation of the cup element such as not to create impedance variations of the detected signal, which can lead to incorrect readings and transductions of the signal itself.

In order to optimize the electrical properties of the cup element according to the configuration just described, it is possible to include that said cup element consists of conductive thermoplastic material, comprising a thermoplastic elastomer inside which particles of conductive material are inserted.

Preferably, such particles are graphite based.

Furthermore, according to a further embodiment the transmission part consists of a wire of conductive material, fixed to the cup element.

A sheath in rigid material is further provided at the connection between the wire and the cup element.

The connection area between the wire and the cup element is particularly critical, as it is a particularly flexible connection, which could cause problems in the signal transmission.

The presence of the sheath instead allows to create a rigid connection point, which does not generate problems for the impedance of the detected signal.

Advantageously, the sheath may consist of a thermo-adhesive sheath, in order to facilitate the application thereof.

Further, the presence of at least one layer of conductive material on the cup element is provided.

The deposition of the layer of conductive material occurs at least in the part of the cup element in contact with the layer of electrolyte material, in order to increase the conductive capacities thereof.

The layer of conductive material has the same capabilities and properties as the cup element in terms of flexibility and softness, so as to allow the deformation of the cup element to be followed by that of the conductive coating, without the latter being able to present cracks or breakages which compromise the conduction of the signal detected by the electrode.

For this reason, the layer of conductive material is made of one or more layers of conductive ink.

Such a configuration allows to increase the electrical conductivity of the electrode, without limiting the flexibility features of the electrode itself.

As anticipated, two solutions can be included to obtain a soft and/or flexible electrode, the application of which does not cause the "skin breakdown" effect.

The first solution, described, was to create a soft conduction part, i.e., the cup element.

The second solution instead includes a combination of electrode flexibility, combined with a decrease in the total weight of the electrode itself.

According to this solution, the electrode object consists of a plurality of branches, of which each branch has a head part and a tail part.

The contact part of the electrode therefore consists of the set of the head parts, while the transmission part consists of the set of the tail parts.

Each branch also has a flexible support element of at least one conductive element, which includes a head terminal and a tail terminal.

Furthermore, the conductive element has at least one head contact and at least one tail contact, respectively arranged at the head terminal and the tail terminal of the flexible support element.

Finally, it is specified that the conductive element consists of a conductive track made of a layer of conductive ink deposited on the flexible support element.

Each branch can therefore be obtained as described in the application WO2018/122824.

The object is to obtain an electrode consisting of flexible parts, in this case both the conduction part and the transmission part are flexible and do not generate pressure forces on the patient's skin.

In addition to flexibility, the peculiarity of the materials forming the electrode according to the configuration just described, confer lightness to the electrode itself, further decreasing the forces acting on the patient's skin.

As will be apparent from an embodiment illustrated and described below, the electrode includes the head terminals in fixed and predetermined positions, in order to be provided in contact with strategic points for detecting the electroencephalographic signal.

However, these predefined positions require the production of transmission parts of different lengths.

Therefore, the dimensions of the electrode, which are necessary to carry out an accurate examination, inevitably clash with flexibility, since during the positioning of the electrode itself, the various branches can become entangled, creating difficulties for the doctor who must perform the positioning.

In order to avoid this condition, the dimensions of the various branches play a fundamental role and in particular, since the length is fixed, the width and thickness are particularly important.

The length of the various branches must be fixed and, in particular, less than a value between 23 and 27 centimetres, preferably 25 centimetres.

This requirement is of fundamental importance in order to be able to use the electrode object of the present invention during a magnetic resonance examination, since, with lengths greater than those indicated, cable charge problems may occur during the magnetic resonance examination.

The best compromise between flexibility of the various branches and practicality of use, taking into account the length constraint, can be achieved by providing a thickness of each branch between 50 micrometres and 200 micrometres.

Regarding the width, it is preferably included that the head contact has a width between 3 and 20 millimetres, while the tail contact has a width between 3 millimetres and 16 millimetres.

The specific and relative positions of the various head contacts and terminals will be apparent from the illustration of a preferred embodiment, however from a general point of view, the branches are arranged in a coplanar manner and so as to converge with the tail parts thereof towards a terminal end.

Furthermore, the branches are arranged symmetrically with respect to a plane perpendicular to the plane of coplanarity.

This configuration refers not to a condition of use, but to an embodiment condition of the electrode object of the present invention.

In fact, during the production of the electrode, the various branches are coplanar with each other, but in the application step, precisely thanks to the flexibility obtained, the branches tend to follow the profile of the patient's skull.

These and other features and advantages of the present invention will become clearer from the following description of some exemplary embodiments illustrated in the attached drawings in which:
figure 1 illustrates a possible embodiment of the electrode object of the present invention;
figures 2a and 2b show two embodiments of an electrode which does not form part of the present invention.

It is specified that the figures attached to the present patent application show some preferred embodiments of the electrode object of the present invention to better understand the advantages and features described.

Such embodiments are therefore to be understood for illustrative purposes only and not limited to the inventive concept of the present invention, namely, to obtain an electrode which can be used for recording the EEG signal in patients, during long-term monitoring, without causing the "skin breakdown" effect on the patient's skin.

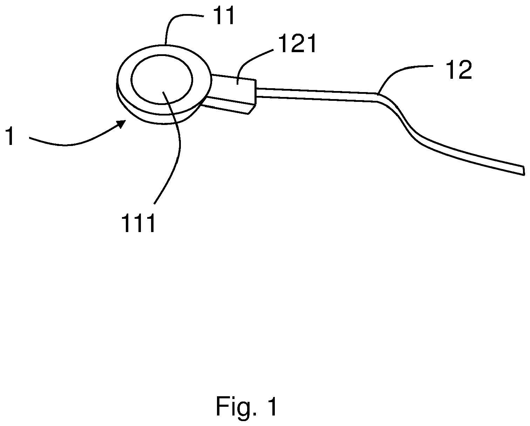

With particular reference to figure 1, a possible embodiment of the electrode 1 for recording electroencephalographic signals is illustrated, comprising a cup element 11 and a transmission part 12.

The cup element 11 has a housing seat 111 adapted to house an electrolyte material, interposed between the cup element 11 and a patient's scalp, for transmitting the signals detected by the patient to the cup element 11.

The cup element 11 is thus arranged with the housing seat 111 facing the patient's scalp, detects the signal of interest and transmits that signal to an external unit via the transmission part, in particular the transmission cable 12.

The external unit is responsible for processing the detected signal and generating the EEG traces.

The cup element 11 must therefore transmit the signal and must have a certain electrical conductivity.

According to the variant illustrated in the figures, the cup element 11 consists of a matrix of rubbery material, inside which matrix particles of conductive material are embedded.

Preferably the cup element 11 consists of conductive thermoplastic material, comprising a thermoplastic elastomer into which graphite-based particles are inserted.

The transmission cable 12 advantageously consists of a wire of metal material coated with an insulating sheath and has two ends, one of which is connected to the cup element 11 and the other connected to a possible external unit, not illustrated in figure 1.

The interface between the cable end 12 and the cup element 11, includes a sheath 121 of rigid material at the connection between the cable 12 and the cup element 11.

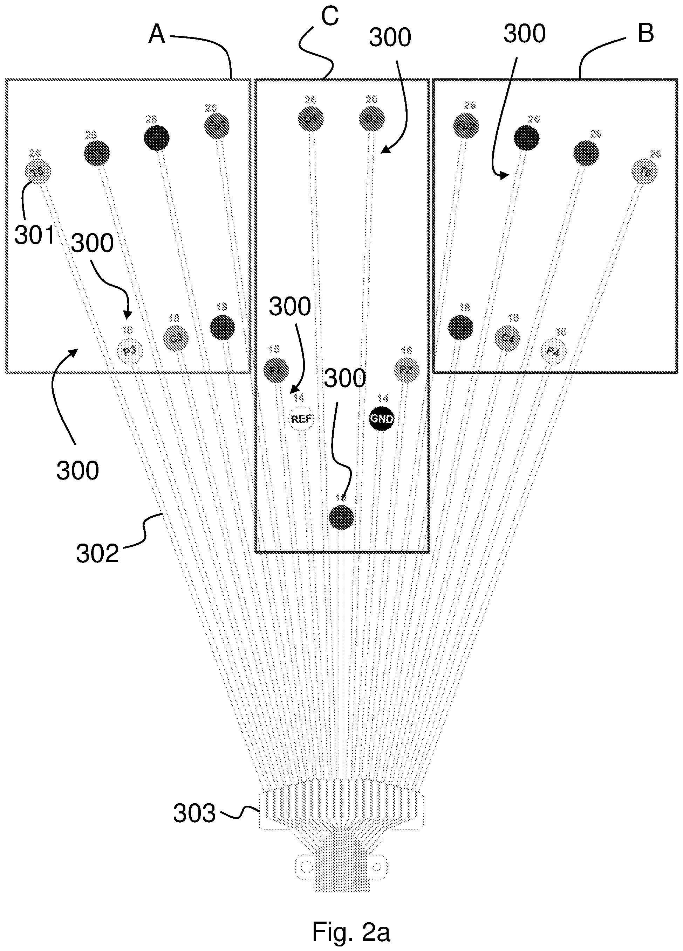

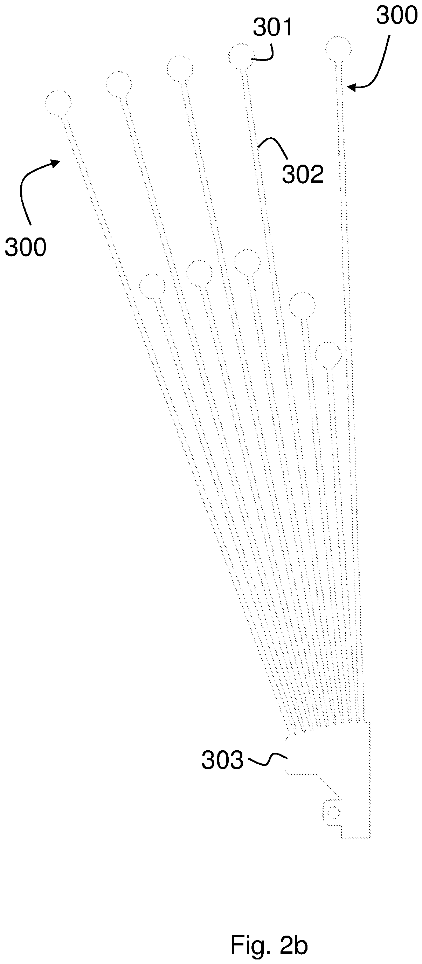

Figures 2a and 2b show a further embodiment of the electrode object of the present invention.

According to such embodiments, the electrode consists of a plurality of branches 300 and each branch has a head portion 301 and a tail portion 302.

Each branch 300 in turn consists of a flexible support element of at least one conductive element, which flexible support element has a head terminal and a tail terminal.

The conductive element has at least one head contact and at least one tail contact, respectively arranged at the head terminal and the tail terminal of the flexible support element.

Furthermore, the conductive element consists of a conductive track made of a layer of conductive ink deposited on the flexible support element.

The conductive element and the flexible support element are not illustrated in figures 2a 2 2b but can be obtained according to one or more of the features described in WO2018/122824.

According to the configuration illustrated in figures 2a and 2b, the head part 301 is adapted to detect the signal from the patient's scalp and transmits this signal to a possible external unit (not illustrated in the figures) through the tail part 302 thereof.

A sponge element used for adhering the head part 301 to the patient's scalp is preferably provided between each head part 301 and the patient's scalp.

The sponge may further comprise the layer of electrolyte material for conducting the signal.

The head portions 301 of each branch 300 are arranged in a predetermined and fixed order, such that, in the condition of use, the head portions 301 are fixed at the points of the patient's scalp where the EEG signals are detected.

Accordingly, the arrangement of the branches 300 is not random, but prior to being arranged on a patient's scalp, the branches 300 are arranged on a common plane, exactly as illustrated in figure 2a.

Furthermore, the branches 300 are arranged with the divergent longitudinal axes thereof in the direction of the head parts 301: a sort of radial arrangement is formed, in which the branches 300 have the ends of the tail parts 302 converging in a common connecting element 303.

The common connecting element 303 may then be connected to a transmission cable for transmitting the signal to an external unit.

Furthermore, the electrode according to figure 2a is symmetrical, with respect to a plane perpendicular to the plane of coplanarity of the branches 300, in particular a plane passing through the longitudinal axis of the central branch 300.

The specific configuration illustrated in figure 2a includes that the branches 300 pass from a width of about 60/70 millimetres, in the area of the connecting element 303, to a width of about 250/260 millimetres in the area of the head parts 301 of the longer branches 300.

In fact, each branch 300 has a specific length, which preferably does not exceed a value between 23 and 27 centimetres, in particular 25 centimetres.

The electrode of figure 2a has five different lengths for the branches 300.

In fact, the symmetrical arrangement of the branches 300 makes it possible to divide the electrode object of the present invention into three groups, each composed of seven branches 300, in particular two side groups A and B and a central group C.

The side groups A and B have the branches 300 with two different lengths, such that the shorter branches 300 are always interposed with the longer branches 300.

The central group C also consists of seven branches 300 and has the central branch 300, of minimum length, interposed between two branches 300 of maximum length and of the same length as the longest branches 300 belonging to groups A and B.

There are then four branches 300 of an intermediate length between the shorter branches and the longer branches of groups A and B.

Preferably the longitudinal axis of each branch 300 has an inclination with respect to the longitudinal axis of the adjacent branch 300 of a value between 1° and 3°.

Figure 2b illustrates a part of an embodiment of the electrode object of the present invention, in particular half of the electrode object of the present invention.

The electrode of figure 2b is made in a manner entirely similar to the electrode of figure 2a with the only difference that the electrode of figure 2a has 21 branches 300, while the electrode of figure 2b, taken entirely, has 20 branches 300.

It follows that the electrode of figure 2b has the same configuration as the electrode of figure 2a, without including the shorter central branch 300.

While the invention is susceptible to various modifications and alternative constructions, some preferred embodiments have been shown in the drawings and described in detail.

It should be understood, however, that there is no intention of limiting the invention to the specific illustrated embodiment but, on the contrary, it aims to cover all the modifications, alternative constructions, and equivalents falling within the scope of the invention as defined in the claims.

The use of "for example", "etc.", "or" refers to non-exclusive non-limiting alternatives, unless otherwise stated.

The use of "includes" means "includes but not limited to", unless otherwise stated.

## Claims

1. Electrode (1) for recording electroencephalographic signals and/or for stimulating patients, comprising a patient contact part and a transmission part (12),
a layer of electrolyte material is further provided which during use is in contact with the patient's skin and interposed between the patient's skin and the contact part,
said contact part consisting of a soft and/or flexible material,
said contact part comprising a cup element (11) adapted to house at least in part said layer of electrolyte material, which cup element (11) consists of a matrix of rubbery material, inside which matrix particles of conductive material are embedded
**characterized in that**
a layer of conductive material is provided, interposed, at least in part, between the cup element and the layer of electrolyte material,
said layer of conductive material consists of one or more layers of conductive ink.

2. Electrode according to claim 1, wherein said cup element (11) consists of conductive thermoplastic material, comprising a thermoplastic elastomer inside which elastomer graphite particles are inserted.

3. Electrode according to claim 2, wherein said transmission part consists of a wire of conductive material (12), which wire (12) is fixed to said cup element (11), a sheath (121) of rigid material being provided at the connection between the wire (12) and said cup element (11).

## Patentansprüche

1. Elektrode (1) zum Aufzeichnen elektroenzephalographischer Signale und/oder zum Stimulieren von Patienten, umfassend einen Patientenkontaktteil und einen Übertragungsteil (12), wobei ferner eine Schicht aus Elektrolytmaterial bereitgestellt ist, die während der Verwendung mit der Haut des Patienten in Kontakt steht und zwischen der Haut des Patienten und dem Kontaktteil eingefügt ist, wobei der Kontaktteil aus einem weichen und/oder flexiblen Material besteht, wobei der Kontaktteil ein Napfelement (11) umfasst, das dazu angepasst ist, die Schicht aus Elektrolytmaterial mindestens zum Teil aufzunehmen, wobei das Napfelement (11) aus einer Matrix aus gummiartigem Material besteht, in die Matrixteilchen aus leitfähigem Material eingebettet sind, **dadurch gekennzeichnet, dass** eine Schicht aus leitfähigem Material bereitgestellt ist, die mindestens zum Teil zwischen dem Napfelement und der Schicht aus Elektrolytmaterial eingefügt ist, wobei die Schicht aus leitfähigem Material aus einer oder mehreren Schichten aus leitfähiger Tinte besteht.

2. Elektrode nach Anspruch 1, wobei das Napfelement (11) aus leitfähigem thermoplastischem Material besteht, das ein thermoplastisches Elastomer umfasst, in das Elastomer-Graphitteilchen eingesetzt sind.

3. Elektrode nach Anspruch 2, wobei das Übertragungsteil aus einem Draht aus leitfähigem Material (12) besteht, wobei der Draht (12) an dem Napfelement (11) befestigt ist, wobei eine Hülle (121) aus starrem Material an der Verbindung zwischen dem Draht (12) und dem Napfelement (11) bereitgestellt ist.

## Revendications

1. Électrode (1) pour l'enregistrement de signaux électroencéphalographiques et/ou la stimulation de patients, comprenant une partie de contact avec le patient et une partie de transmission (12), une couche de matériau électrolytique est en outre prévue qui, lors de l'utilisation, est en contact avec la peau du patient et intercalée entre la peau du patient et la partie de contact, ladite partie de contact étant constituée d'un matériau souple et/ou flexible, ladite partie de contact comprenant un élément de coupelle (11) adapté pour loger au moins en partie ladite couche de matériau électrolytique, élément de coupelle (11) qui est constitué d'une matrice de matériau caoutchouteux, à l'intérieur de laquelle des particules de matrice de matériau conducteur sont incorporées, **caractérisée en ce qu'**une couche de matériau conducteur est prévue, intercalée, au moins en partie, entre l'élément de coupelle et la couche de matériau électrolytique, ladite couche de matériau conducteur étant constituée d'une ou plusieurs couches d'encre conductrice.

2. Électrode selon la revendication 1, dans laquelle ledit élément de coupelle (11) est constitué d'un matériau thermoplastique conducteur, comprenant un élastomère thermoplastique à l'intérieur duquel des particules de graphite élastomère sont insérées.

3. Électrode selon la revendication 2, dans laquelle ladite partie de transmission consiste en un fil de matériau conducteur (12), fil (12) qui est fixé audit élément de coupe (11), une gaine (121) de matériau rigide étant prévue au niveau de la connexion entre le fil (12) et ledit élément de coupe (11).
